(19) Europäisches Patentamt
European Patent Office
Office européen des brevets

(11) **EP 2 367 547 B1**

(12) **EUROPEAN PATENT SPECIFICATION**

(45) Date of publication and mention
of the grant of the patent:
**30.07.2014 Bulletin 2014/31**

(51) Int Cl.:
**A61K 31/198** (2006.01)    **A61P 25/26** (2006.01)

(21) Application number: **09775374.3**

(22) Date of filing: **11.12.2009**

(86) International application number:
**PCT/SE2009/051407**

(87) International publication number:
**WO 2010/068173 (17.06.2010 Gazette 2010/24)**

(54) **L-LYSINE FOR IMPROVING NORMAL COGNITIVE FUNCTIONS**

L-LYSIN ZUR VERBESSERUNG NORMALER KOGNITIVER FUNKTIONEN

L-LYSINE POUR AMÉLIORATION DES FONCTIONS COGNITIVES NORMALES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **12.12.2008 SE 0802556**

(43) Date of publication of application:
**28.09.2011 Bulletin 2011/39**

(73) Proprietor: **Cognite AB**
**411 24 Göteborg (SE)**

(72) Inventor: **KLAMER, Daniel**
**411 24 Göteborg (SE)**

(74) Representative: **Valea AB**
**Anna Lindhs Plats 4**
**211 19 Malmö (SE)**

(56) References cited:
**EP-A1- 1 707 194       WO-A1-02/07768
WO-A1-2008/103939**

• **LACZI F ET AL: "Effects of lysine-vasopressin and 1-deamino-8-D-arginine-vasopressin on memory in healthy individuals and diabetes insipidus patients." PSYCHONEUROENDOCRINOLOGY 1982, vol. 7, no. 2-3, 1982, pages 185-193, XP24386358 ISSN: 0306-4530**
• **Anonymous: "Lysine" Vita Viva - Info, [Online] 10 August 2008 (2008-08-10), pages 1-3, XP002566126 Retrieved from the Internet: URL: http://www.vitaviva-info.com/en/Health /Health_ Products_Details.29.aspx> [retrieved on 2010-01-29]**

## Description

### *Technical Field*

The present invention relates to the use of L-lysine to improve cognitive function in normal healthy subjects.

### *Background of the invention*

In life, we experience situations daily when we wished we had better cognitive functions. Vast interest in finding ways to improve our normal cognitive abilities has inspired a new invention.

The present invention improves cognitive function in normal subjects. These improvements are achieved by administration of L-lysine.

L-lysine is an essential and basic amino acid (i.e., carries a positive net charge at physiological pH) with a high nutritional value. It is required for normal growth and development and, is readily absorbed from the intestine and metabolized by the liver.

High levels of L-lysine are found in muscle. Increased intake of L-lysine is more readily distributed to muscle tissue than to other parts of the body, such as blood. Studies have been performed using L-lysine supplementation for treating conditions such as recurrent herpes simplex infection and osteoporosis. Being an essential amino acid, L-lysine has very good oral bioavailability and easily penetrates the blood-brain barrier.

The only reported adverse effects of L-lysine treatment in humans are transient gastrointestinal problems in a few patients. Based on published studies, long-term addition of 6 g of L-lysine to the daily diet is regarded to be safe. In short-term human studies, doses as high as 40 g/day have been used. The only reported adverse effects were abdominal cramps and transient diarrhea that resolved as the dose was decreased. Long-term studies on L-lysine administration (up to 2 years) have not revealed any adverse effects.

Previously, it has been demonstrated that L-lysine can restore cognitive impairments, as measured by how the brain filters information pre-attentively, in mice. These cognitive deficits were induced by the schizophrenomimetic substance phencyclidine (Pålsson et al, Psychopharmacology (Berl). 2007 May; 192 (1):9-15). Interestingly, a recent pilot study suggests that adjunctive treatment with L-lysine (6 g/day) possibly can improve some symptoms in patients with schizophrenia.

A common misconception is that improving cognitive defects in an ill person is equivalent to enhancing cognitive abilities in a typically healthy person. This may be a fine point, but is key in understanding the technical effect of using L-lysine in a healthy person. For example, an antibiotic would not be useful in an uninfected person, just as L-lysine was not known to previously improve memory and cognition in healthy people.

Importantly, L-lysine has the ability to improve normal cognitive functions, as this invention claims. This differs from previous studies where L-lysine restored some symptoms in patients suffering from an underlying disease, or cognitive impairments in laboratory disease models. As another example, antipsychotic treatments can improve cognitive impairments of schizophrenia, as well as reduce some symptoms in this patient group. However, no one would claim that antipsychotic treatment would improve baseline cognitive performance in healthy individuals.

Hence there is a difference in improving an impairment caused by an underlying pathophysiology as compared to improving a state from a natural baseline. Another example would be that, if a person has a sore throat caused by a Streptococcal infection, antibiotic medication would help against this disease state. However, taking antibiotics if you are healthy and do not have any symptoms associated with Streptococci, antibiotics would not make you, or your throat better. In fact, taking antibiotics could be harmful for a normal functional person.

An example from the neuropsychiatric field is the selective serotonin reuptake inhibitors or SSRI. This class of pharmaceutical agents has proven highly effective in the treatment of e.g. depression and anxiety related disorders but have no positive effects on mood in healthy individuals. This division between effects in a disease state versus effects on normal function is a well-known fact in the neuropsychiatric field. Therefore a person skilled in the art cannot assume that a beneficial effect in a disease or disease model equals a beneficial effect in a healthy individual. It would be similar to assuming that an invention that can repair a broken car would also make a fully functional car go faster.

In fact, cognition-enhancing compounds used in Alzheimer's disease constitute another example of the dissociation between treating deficits in a patient population and enhancing baseline cognition in healthy volunteers. Both the NMDA antagonist Memantine and the acetylcholinesterase inhibitor Donezepil enhance cognition in patients with Alzheimer's disease, but have no or detrimental effects on cognition in healthy volunteers. These findings clearly underscore the difference in approach between improving normal versus impaired cognition and suggest that extrapolation of a given drug effect from one population to the other is speculative at the best.

Hence it is not obvious for a person skilled in the art to make the assumption that L-lysine has the possibility to improve cognitive performance from a natural baseline, simply because it is has been claimed to alleviate cognitive impairments associated with different diseases or deficiencies.

As defined in the present application a healthy individual is somebody who does not have an underlying disease, such as schizophrenia, ADHD, Alzheimer's disease, other dementia-related diseases and/or other diseases that could cause cognitive impairments. Moreover, a healthy individual does not suffer from deficiency states or related deficiency symptoms. Signs and symptoms of L-lysine deficiency include fatigue, nausea, dizziness, and declined concentration.

WO 02/07768 and US2004005311 disclose dietary supplements, which are claimed to alleviate dementia-related symptoms. These supplements include a core of L-lysine and bromelain plus a range of different substances and vitamins, e.g., lycopene, Vitamin C, Vitamin E and folic acid. However, this composition comprises at least both L-lysine and bromelain and alleviates impairments that already exist with a combination of several compounds.

The website VitaViva, dated 2008-08-10 (www.vitaviva-info.com) mentions that L-lysine improves concentration. However, the context is that L-lysine can improve concentration in people suffering from L-lysine deficiency, since deficiency symptoms include tiredness and dizziness. Hence, there is no mentioning of that L-lysine would improve concentration in healthy individuals.

WO 95/16661 claims that 4,7,10,13,16,19-cis-docosahexaenoic acid (DHA) salts with basic amino acids, in particular arginine and lysine, have favorable therapeutically and technological characteristics compared with the starting acid. However, nothing in WO 95/16661 suggests that the amino acid L-lysine by itself improves cognitive performance from a natural baseline.

EP0198508 states that L-Phosphoserine amino acidic derivates with L-arginine and/or L-lysine may constitute a pharmacological composition in order to treat memory deficiencies, intellection deficiencies and mental deterioration. Hence, L-lysine is part of another molecule, L-Phosphoserine salt, which could be used to therapeutically treat cognitive impairments.

US2007036870 claims the use of a bioavailable iron compound and a bioavailable zinc compound in a weight ratio of at least 2:1, and at least one B vitamin, in the manufacture of an edible composition, for use in aiding the cognitive development or cognitive performance of humans having an age of up to 18 years. L-lysine is mentioned in the context that it can be one of many chelating ligands in order to increase the bioavailability for the active compounds.

WO9833498 claims that administering of therapeutically effective amount of Breflate or a Breflate derivative can treat a mammal suffering from cognitive dysfunction as well as enhancing cognitive functions in a mammal. Residues to the above compounds could include amino acids such as glycine, alanine, leucine, isoleucine, valine, phenylalanine, proline, lysine and arginine. Again there is no evidence that the amino acid L-lysine by itself improves cognitive performance from a natural baseline.

Taken together, none of the above documents or other sources of public information anticipate the surprising effect of L-lysine regarding improved cognitive performance on healthy individuals.

## Summary of the invention

The present invention improves cognitive function in normal subjects. These improvements are achieved by L-lysine (L-lysine monhydrochloride).

A first aspect of the invention relates to a composition comprising L-lysine for the improvement of cognitive functions and performances in normal healthy mammals.

In another embodiment of the invention the mammal is a human.

In another embodiment of the invention the cognitive function is pre-attentive and attentive information processing or attentional capacity.

In yet another embodiment of the invention the cognitive function is the memory, including the long-term memory, short-term memory and the working memory.

In another embodiment of the invention the cognitive function is the executive function or reasoning.

In a further embodiment of the invention the composition is administered daily.

In another embodiment of the invention the effective dose of L-lysine is 500 - 40 000 mg/day, preferably between 1000 - 30 000 mg/day, more preferably between 2000 - 25 000 mg/day and the most preferred dose ranges between 3000 - 20 000 mg L-lysine per day.

In another embodiment of the invention the composition is administered orally.

In a further embodiment of the invention the composition is formulated in a form or shape of the group comprising the following forms: tablets, pills sachets, vials, hard or soft capsules, aqueous or oily suspensions, aqueous or oily solutions, emulsions, powders, granules, syrups, elixirs, lozenges, reconstitutable powders, liquid preparations, creams, troches, hard candies, sprays, liquid aerosols, dry powder formulations, HFA aerosols or organic or inorganic acid addition salts.

In another embodiment of the invention the composition further comprises a carrier or excipient.

In another embodiment of the invention the composition is administered as a single dose or by multiple doses daily.

A second aspect of the invention relates to the use of L-lysine for the manufacture of a composition for the improvement of cognitive functions and performances in normal healthy mammals.

In another embodiment of the invention the mammal is a human.

In another embodiment of the invention the cognitive function is pre-attentive and attentive information processing.

In another embodiment of the invention the cognitive function is attentional capacity.

In another embodiment of the invention the cognitive function is the memory, including the long-term memory, short-term memory and the working memory.

In another embodiment of the invention the cognitive function is the executive function.

In another embodiment of the invention the cognitive function is reasoning.

In another embodiment of the invention the composition is administered daily.

In another embodiment of the invention the effective dose of L-lysine is 500 - 40 000 mg/day, preferably between 1000 - 30 000 mg/day, more preferably between 2000 - 25 000 mg/day and the most preferred dose ranges between 3000 - 20 000 mg L-lysine per day.

In another embodiment of the invention the composition is administered orally.

In another embodiment of the invention the composition is formulated in a form or shape of the group comprising the following forms: tablets, pills sachets, vials, hard or soft capsules, aqueous or oily suspensions, aqueous or oily solutions, emulsions, powders, granules, syrups, elixirs, lozenges, reconstitutable powders, liquid preparations, creams, troches, hard candies, sprays, liquid aerosols, dry powder formulations, HFA aerosols or organic or inorganic acid addition salts.

In another embodiment of the invention the composition further comprises a carrier or excipient.

In another embodiment of the invention the composition is administered as a single dose or by multiple doses daily.

A third aspect of the invention relates to a method for improving cognitive functions and performances in normal healthy mammals, wherein an effective amount of a composition comprising L-lysine is administered to a mammal.

### Definitions

The terms "cognition", "cognitive function" and "cognitive performance" as used herein are interchangeable and refer to a range of cognitive domains such as for example:

- Pre-attentive and attentive information processing (including processes with both intrinsic and extrinsic stimuli)

- Attentional capacity (the process of selectively concentrating on one aspect of the environment while ignoring other stimuli)

- Memory (encoding, storage, retrieval)

- Short-term memory (memory that involves recall of information for a relatively short time)

- Long-term memory

- Working memory (temporarily storing and manipulating information)

- Reasoning (cognitive process of looking for reasons for beliefs, conclusions, actions or feelings)

- Executive functions (dealing with novelty, planning and implementation, monitoring performance, vigilance, inhibition of task irrelevant information)

- A conscious intellectual act

- Intellectual capacity

However, it should be understood by the person skilled in the art that the list of cognitive functions and/or cognitive performances mentioned above does not exclude other forms of cognitive functions.

### Brief description of the drawings

Figures 1A and B shows that L-lysine improves cognitive function in a dose-related manner by assessing how the brain filters information pre-attentively.

Figure 2 show that L-lysine improves cognitive function by assessing non-associative learning.

Figure 3A and B shows that L-lysine improves cognitive function by assessing learning and memory formation.

### Detailed description of the invention

In the present invention it is shown that L-lysine improves natural and baseline cognitive performances.

The following experiments (example 1, 2 and 3) demonstrate that L-lysine (L-lysine monohydrochloride) can enhance baseline cognitive performance in normal healthy mammals.

Example 1 - Pre-attentive information processing

One way to elucidate how substances affect our cognitive function is to measure how the brain processes information. Pre-attentive information processing is thought to be important for selective and efficient processing of sensory information and for coherent cognitive operations, and thus for memory and cognitive functions. Pre-attentive information processing can be assessed by using the prepulse inhibition (PPI) of acoustic startle response model. One major advantage of the PPI model is that it is a translational behavior. Hence, it can be studied in a variety of species, from rodents to humans, and provides unique opportunities for cross species explorations of how the brain processes information pre-attentively. PPI is the reduction in reflex response to an intense stimulus when this stimulus is immediately preceded (30-500 ms) by a weak prestimulus. The presentation of the prestimulus, although not strong enough to elicit a measurable response by itself, evokes a short lasting sensory gating process in the brain, which is manifested by the attenuated response to the successive intense stimulus. PPI is hypothesized to reflect the ability of the central nervous system to filter out distracting and irrelevant stimuli before it reaches the cortex. An increased, i.e. improved, PPI could result in better inhibitory mechanisms that filter or gate intrinsic and extrinsic stimuli prior to higher order processing. Hence, improved pre-attentive information processing could result in better cognitive performances. Furthermore, PPI and other cognitive functions are regulated by the same cortico-striato-pallido-thalamic circuitry and association between these different cognitive domains has been shown.

Male NMRI mice (Charles river, Germany, 25-35 g) were used in examples 1, 2 and 3. The animals were housed nine per cage in a colony room under constant temperature ($20 \pm 1°C$) and humidity ($50 \pm 5\%$). Animals were allowed to acclimatize for at least one week prior to behavioral testing. The daylight cycle was maintained artificially (lights on from 0600 to 1800 hours) and behavioral experiments were conducted during the light phase. The Ethics Committee for Animal Experiments, Göteborg, Sweden, approved all experimental procedures used in the present study.

Acoustic startle was recorded using a MOPS 3 startle response recording system (Metod och Produkt Svenska AB, Sweden). The animals were placed in small plexi glass cages (10 x 5.5 x 6 cm) that were suspended at the top of a piston. A movement of the animal in the cage caused a displacement of the piston, the acceleration of which was registered by a piezo-electric accelerometer. This signal was sampled and digitized by a microcomputer that also controlled the delivery of acoustic stimuli. Startle amplitude was defined as the maximum signal amplitude occurring 8-30 ms after the startle-eliciting stimulus. Four cages were used simultaneously and each cage was housed in a dimly lit and sound attenuated cabinet (52 x 42 x 38 cm). The cages were calibrated for equal sensitivity prior to testing and a mouse was always tested in the same cage at subsequent tests. The acoustic stimuli consisted of white noise, which was delivered by two high-frequency loudspeakers built into the ceiling of the cabinet.

Each test session started with an 8-min adaptation period containing only white background noise at 65 dB (A). Startle pulse was set to 105 dB (A) and prepulse intensities to 3, 9 and 12 dB (A) above background noise. Duration of acoustic stimuli was set to 20 ms for both prepulses and startle pulses and interstimulus interval (ISI) was set to 40 ms. Following the adaptation period the animals were subjected to a series of 5 startle pulse-alone trials that were omitted from the analysis since they only served to accommodate the animals to the sudden stimulus onset. The animals were then subjected to a pseudo-randomized combination of 3 prepulse-alone trials for each prepulse intensity, 45 pulse-alone trials and 15 prepulse+pulse trials for each prepulse intensity. Trials were separated by 5-15 s intervals.

All mice were first subjected to a pre-test to ensure that they expressed basal startle reactivity. Thereafter, the animals were subjected to matching test and based on the results of this test the animals were divided into four treatment groups with comparable basal PPI and startle reactivity. During three consecutive days (day 1 to 3) the groups received, once daily, either saline (10 ml/kg, n=16) or L-lysine (500 mg/kg, n=16; 1000 mg/kg, n=15; or 2000 mg/kg, n=17). PPI was tested on day 1 and day 3 with administration of saline/L-lysine 25 min before presentation of the first startle stimuli. This design was used to evaluate both the acute and sub-chronic effects of L-lysine treatment. A final PPI test was performed at day 9, hence when the animals have been without any L-lysine treatment for 6 days. The final test was performed to evaluate if the effects of L-lysine were acute or persisted over time.

The mean response amplitude for pulse-alone trials (P) was calculated for each mouse and test. This measure was used in the statistical analysis to assess drug-induced changes in acoustic startle response. The mean response amplitude for prepulse-pulse trials (PP) was also calculated and used to express the percent PPI according to the following formula:

$$\text{Prepulse inhibition (\%)} = 100 - [(PP/P) * 100]$$

Using this formula, a 0% value denotes no difference between pulse-alone and prepulse-pulse response amplitudes and consequently no PPI. Statistical analysis for the experiments was performed using a two-way mixed model ANOVA with test day as swithin subjects factor and dose as between subject factor. Two tailed levels of significance were used and $p < 0.05$ was considered statistically significant.

The results demonstrate a significant effect of L-lysine treatment on day 1 and day 3 ($F(1, 44)=94.9$; $P < 0.05$) at a prepulse level of 9 dB over background noise. The doses 500 mg/kg and 1000 mg/kg improved the PPI response in a dose related manner, while the highest dose L-lysine, 2000 mg/kg, did not affect PPI significantly. These results point towards an inverted U-shaped response curve regarding cognitive performance and levels of L-lysine. The cognitive performance improved with increased levels of L-lysine, but only up to a point. When levels of L-lysine became too high, performance decreased. An inverted U-shaped response curve is not uncommon when it comes to cognitive performances.

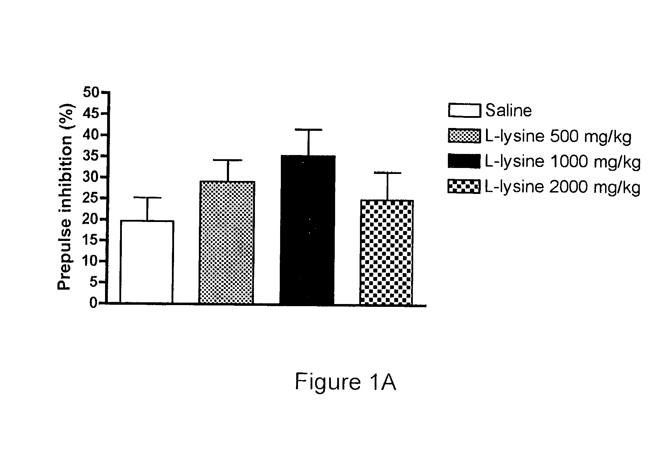

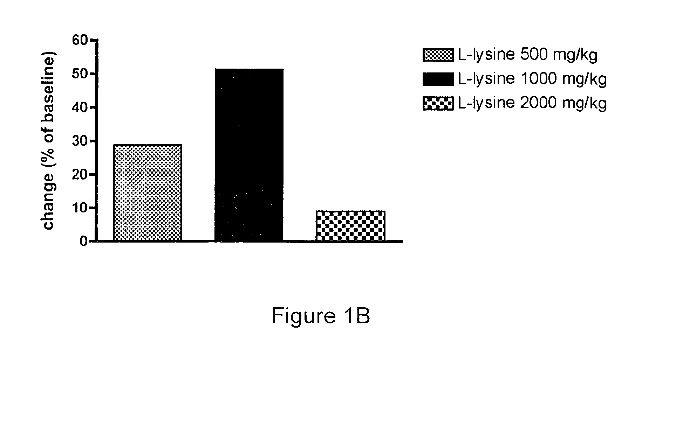

In figure 1A, the effects of L-lysine (0, 500, 1000, and 2000 mg/kg, intraperitoneally) are shown after acute administration (day 1). In figure 1B data is presented as absolute change in PPI, at day 1, compared to what the different treatment groups had in the matching test. Figure 1B demonstrates that the capacity to filter information pre-attentively was increased with 28.7% in the group that was treated with L-lysine 500 mg/kg and 51.3% in the group administrated with L-lysine 1000 mg/kg and with 9.0% in the L-lysine 2000 mg/kg group.

The results also demonstrated that there was no significant effect of test day (comparing the results of PPI at day 1 with day 3). Hence, the beneficial effects on PPI function do not increase or decrease with repeated L-lysine administration.

Moreover, the final PPI test at day 9, after a 6-day washout period, did not demonstrate any significant effects of L-lysine treatment. Thus, the beneficial effects of L-lysine disappear if the administration terminates.

Finally, L-lysine administration did not affect the pulse-alone response. Hence, L-lysine doesn't seem to have any sedative properties.

In summary, these results demonstrate that administration of L-lysine improves normal cognitive functions, measured by how the brain filters information pre-attentively in the PPI model. Administration of L-lysine enhances cognitive performance directly and these improvements persist if L-lysine is continuously administrated. However, when administration is interrupted the cognitive performance returns to baseline.

Example 2 - Non-associative learning

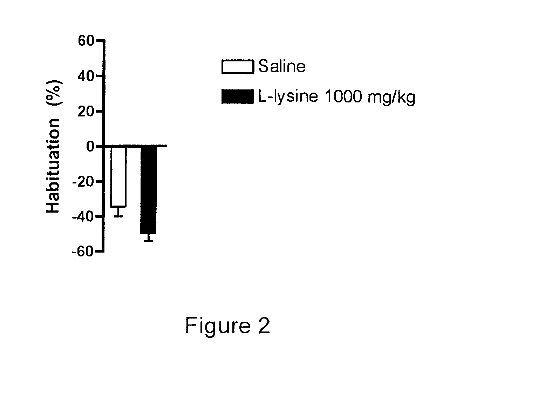

The aim was to investigating the effects of L-lysine administration in a model measuring non-associative learning. As predicted from the study measuring pre-attentive information processing (example 1), mice administrated L-lysine 1000 mg/kg increased the capacity to screen out irrelevant stimuli and thus demonstrated an improved learning (see figure 2). The model used to measure non-associative learning was habituation of acoustic startle response. Habituation refers to the decrease in response to repeated presentation of identical stimuli and the model used for this test is also translational in nature. Since habituation is a measure of how the brain filter outs irrelevant stimuli and focus selectively on important stimuli, it is thought to be a prerequisite for other forms of learning and cognitive functions.

Acoustic startle was recorded using a MOPS 3 startle response recording system (see example 1).

The mice were tested for habituation according to the following protocol: The mice were first subjected to a pre-test. The mice were placed in the startle cages in the enclosure for a 10 min accommodation period consisting of 65 dB (A) background noise. After the habituation period they were presented with 20 pulse-alone trials. The time interval between the trials was always 10 s. Pulse intensity was set to 105 dB (A) and the duration of each pulse was 20 ms. After the pre-test the mice were matched and randomized into homogenous groups with their mean startle response and SEM as reference (L-lysine 1000 mg/kg; n=12. Saline 10 ml/kg; n=12).

The mice used in the habituation test were again placed in the startle cages in the enclosures for a 10 min accommodation period consisting of 65 dB background noise. After this period they were presented with 121 pulse-alone trials. The time interval between the trials was always 10 s. Pulse intensity was set to 105 dB and the duration of each startle was 20 ms.

Administration of saline/L-lysine intraperitoneally was 25 min before presentation of the first startle stimuli. The first startle pulse response was omitted from statistical analysis since it was too variable to achieve significance. Hence 120 startle pulse trials were used in the analysis. The 120 pulses were divided into 6 blocks, each block containing 20 pulses. Habituation, the change in mean response amplitude over time, was calculated using the formula:

$$\text{Habituation (\%)} = [(\text{block number x/block number 1}) \times 100] - 100$$

Using this formula, a 0% value denotes no difference between in startle response amplitude between block number 1 and other subsequent blocks and consequently no habituation. Negative values indicate a decreased response over time, i.e., a habituation of the acoustic startle response.

In figure 2 the effects of acute administration of L-lysine 1000 mg/kg on the habituation of the acoustic startle response are shown. L-lysine significantly, at block 6 (unpaired t-test, t=2.099, df=22, p<0.05), improves the brain capacity to filter out irrelevant information.

Example 3 - Learning and memory formation

There are several learning and memory tasks that are appropriate for study in rodents. One widely used is the novel object recognition test. The test involves exposing the rodent to two identical objects for a brief period of time (the introductory session). After a delay the rodent is placed back to the arena (the recognition session) with one of the familiar objects it encountered in the introductory session and an additional novel object. Rodents typically spend more time exploring the novel object (if they have a memory from the introductory session). Task difficulty can be increased by e.g. prolonging the delay between sessions or increasing the number of objects presented. Some of the benefits with the object recognition model are that it is well characterized and not based on reward or punishment. The object recognition test is based on the animals' innate behavior to investigate their environment. Furthermore, very similar aspects of memory formation (involving encoding, consolidation and retrieval of information) can be assessed in human using the Brief Visual Memory Task - Revised (BVMT-R). Hence the object recognition test is a valid and translational model to investigate the effects of different substances on memory.

The aim was to investigating the effects of L-lysine administration in the object recognition model.

The experimental setup for object recognition consisted of a box-shaped Plexiglas arena with a floor area of 40x40 cm. The illumination at the floor of the arena was 5-7 lux and the arena was videotaped from above with a digital video camera.

Objects to discriminate were a green wooden star-shaped cylinder (h, Ø: 6 x 3.5 cm) and a drinking glass (h, Ø: 5.5 x 5.8 cm). Objects were placed 14 cm apart and 11 cm from the closest wall.

The object exploration time was manually scored from the videotapes. Object exploration was defined as mice sniffing or touching the object with nose and/or forepaws.

The day before the object recognition test the animals were acclimatized to the arena for 5 min. On the day of the experiment the animals were transferred to the laboratory, marked and weighed and thereafter left to acclimate to the environment for approximately 2.5 h before testing started. Administration of L-lysine 1000 mg/kg (n=17) or saline 10 ml/kg (n=19) was given intraperitoneally 30 min before the introductory session.

In the first, i.e. the introductory, session the mice were placed in the middle of the arena and presented with two identical objects, A1 and A2, during 5 min (the green wooden star-shaped cylinder). After a 90 min delay in the home cage, the mice were again placed in the middle in the same arena as earlier and presented with two objects, the old, familiar A1, and a new object B1 (the drinking glass) during 5 min (the recognition session). Object A2 was always the one that was replaced. Between each mouse and session the objects were cleaned with 10% ethanol.

Data are expressed as means and standard errors of the mean (S.E.M.). If object exploration time during the introductory session was <5 s/object the mice were discarded from the sample.

A discrimination index was calculated for each animal: Tn/(Tf+Tn)*100

Tn=time spent exploring the novel object (B1).

Tf=time exploring the object (A1) under recognition.

The discrimination index was expressed in percentages. An animal spending equal time exploring the two objects during a session will receive a discrimination index of 50%, while an animal exploring the novel object a longer time compared with the familiar will receive a value >50%.

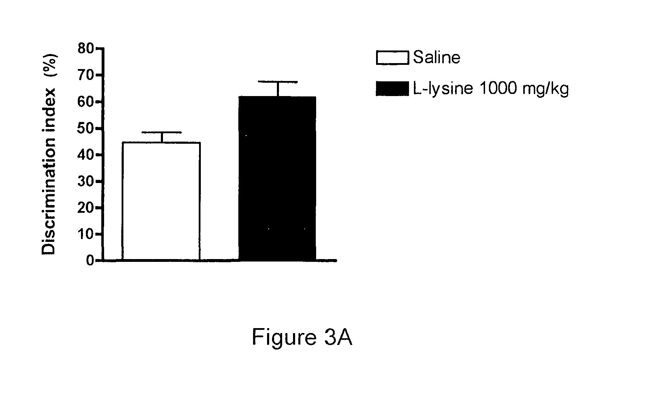

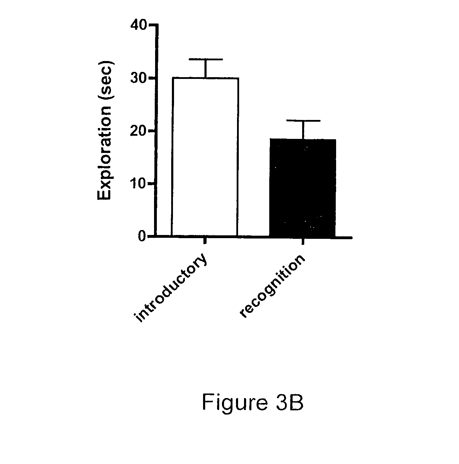

There was no difference between L-lysine (1000 mg/kg) and saline (10 ml/kg) treatments in time exploring the two similar objects during the introductory session (two-way mixed model ANOVA with treatment as between subject factor and object as within subject factor), indicating that there was no preference for a certain position of the objects in the arena as well as administration of L-lysine did not influence the explorative behavior. Figure 3A shows that mice that have been administrated L-lysine spent significantly more time exploring the novel object compare to saline treated mice, as demonstrated by the discrimination index during the recognition session (unpaired t-test, t=2.580, df=34, p<0.05). Moreover, in figure 3B administration of L-lysine also resulted in significantly less exploration of the familiar object during the recognition session compare the time the object was explored during the introductory session (unpaired t-test, t=2.273, df=32, p<0.05), while there was no significant difference in the control group (unpaired t-test, t=1.463, df=36, p=ns).

The main finding was that administration of L-lysine increased object recognition compared to saline treatment. The better learning and memory was demonstrated by decreased exploration to the familiar object during recognition session compared to the introductory session. Furthermore, the L-lysine treated animals also explored the novel object more compared to the familiar object during the recognition session. Taken together, this demonstrates that administration of L-lysine improves cognitive performance from a natural and non-pathological non-deficiency baseline.

L-lysine could be used as a supplement in food and drinks, e.g. in bread, cereals, cookies, chips, bars, candies, pastries, ice cream, gum, juices, sodas, energy drinks, yogurt drinks as well as in animal foods and as a salt substitute.

Preferably the L-lysine is supplemented in a free form, hence not part of e.g. a peptide. The compound of the present invention may be isolated in any level of purity by standard methods and purification can be achieved by conventional means known to those skilled in the art, such as distillation, recrystallization and chromatography. L-lysine may be manufactured by a fermentation process using carbohydrate sources.

The compound of the invention may be administered alone or in combination with acceptable carriers or diluents, and such administration may be carried out in single or multiple doses.

The dose range, for oral administration of L-lysine, is 500 - 40 000 mg/day. The dose is preferably between 1000 - 30 000 mg/day, more preferably between 2000 - 25 000 mg/day. The most preferred oral dose ranges between 3000 - 20 000 mg/day which may be administered as a single dose or by multiple doses daily. Compositions may, for example, be in the form of tablets, pills sachets, vials, hard or soft capsules, aqueous or oily suspensions, aqueous or oily solutions, emulsions, powders, granules, syrups, elixirs, lozenges, reconstitutable powders, liquid preparations, creams, troches, hard candies, sprays, liquid aerosols, dry powder formulations, HFA aerosols or organic or inorganic acid addition salts.

The compositions of the invention may be in a form suitable for administration through oral, parenteral, topical, dermal, subcutaneous, intravenous, intramuscular, buccal, rectal or vaginal routes, or for administration by inhalation or insufflation (e.g. nasal, tracheal, bronchial) routes. However, oral administration is the preferred route of administration.

Depending upon the subject the route of administration, the compositions may be administered at varying doses.

The compound of the present invention may be able to form salts with acceptable acids or bases.

Suitable acid addition salts of the compound of the present invention include but are not limited to those formed with acceptable salts such as toluensulfonate, methanesulfonate, fumarate, hydrochloride, hydrobromide, hydroiodide, nitrate, acetate, lactate, citrate, acid citrate, tartrate, bitartrate, aliphatic, alicyclic, aromatic or heterocyclic carboxylate, succinate, maleate, fumarate, gluconate, glycolate, saccharate, ascorbate, acetate, propionate, benzoate, pyruvate, pamoate [i.e., 1,1'-methylene-bis-(2-hydroxy-3-naphthoate)], phosphate, acid phosphate, sulphate or bisulfate salts.

It is also to be understood that compound of the present invention can exist in solvated as well as unsolvated forms such as, e.g, hydrated forms.

For oral, buccal or sublingual administration, the compound of the present invention may be combined with various excipients. Solid preparations for oral administration often include binding agents (for example syrups and sugars, acacia, gelatin, sorbitol, tragacanth, polyvinylpyrrolidone, sodium lauryl sulphate, pregelatinized maize starch, hydroxypropyl methylcellulose, lactose, starches, modified starches, gum acacia, gum tragacanth, guar gum, pectin, wax binders, microcrystalline cellulose, methylcellulose, carboxymethylcellulose, hydroxypropyl methylcellulose, hydroxyethyl cellulose, hydroxypropyl cellulose, copolyvidone and sodium alginate), disintegrants (such as starch and preferably corn, potato or tapioca starch, alginic acid and certain complex silicates, polyvinylpyrrolidone, sucrose, gelatin, acacia, sodium starch glycollate, microcrystalline cellulose, crosscarmellose sodium, crospovidone, hydroxypropyl methylcellulose and hydroxypropyl cellulose), lubricating agents (such as magnesium stearate, sodium lauryl sulfate, talc, silica polyethylene glycol waxes, stearic acid, palmitic acid, calcium stearate, carnuba wax, hydrogenated vegetable oils, mineral oils, polyethylene glycols and sodium stearyl fumarate) and fillers (including high molecular weight polyethylene glycols. lactose, sugar, calcium phosphate, sorbitol, glycine magnesium stearate, starch, glucose, lactose, sucrose, rice flour, chalk, gelatin, microcrystalline cellulose, calcium sulphate, xylitol and lactitol). Such preparations may also include preservative agents and anti-oxidants.

Liquid compositions for oral administration may be in the form of, for example, emulsions, syrups, or elixirs, or may be presented as a dry product for reconstitution with water or other suitable vehicle before use. Such liquid compositions may contain conventional additives such as suspending agents (e.g. sorbitol, syrup, methyl cellulose, hydrogenated edible fats, gelatin, hydroxyalkylcelluloses, carboxymethylcellulose, aluminium stearate gel, hydrogenated edible fats) emulsifying agents (e.g. lecithin, sorbitan monooleate, or acacia), aqueous or non-aqueous vehicles (including edible oils, e.g. almond oil, fractionated coconut oil) oily esters (for example esters of glycerine, propylene glycol, polyethylene glycol or ethyl alcohol), glycerine, water or normal saline; preservatives (e.g. methyl or propyl p-hydroxybenzoate or sorbic acid) and conventional flavouring, preservative, sweetening or colouring agents. Diluents such as water, ethanol, propylene glycol, glycerin and combinations thereof may also be included.

Other suitable fillers, binders, disintegrants, lubricants and additional excipients are well known to a person skilled in the art.

The compound of the invention may also be administered in a controlled release formulation. The compounds are released at the required rate to maintain constant activity for a desirable period of time. Such dosage forms provide a supply of a drug to the body during a predetermined period of time and thus maintain drug levels in the therapeutic range for longer periods of time than conventional non-controlled formulations. The compound may also be formulated in controlled release formulations in which release of the active compound is targeted. For example, release of the compound may be limited to a specific region of the digestive system through the pH sensitivity of the formulation. Such formulations are well known to persons skilled in the art.

The active compounds may be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

**Claims**

1. Use of a composition comprising L-lysine for the improvement of cognitive functions and performances in normal healthy mammals.

2. Use according to claim 1, wherein the mammal is a human.

3. Use according to claim 1, wherein the cognitive function is pre-attentive and attentive information processing.

4. Use according to claim 1, wherein the cognitive function is attentional capacity.

5. Use according to claim 1, wherein the cognitive function is the memory, including the long-term memory, short-term memory and the working memory.

6. Use according to claim 1, wherein the cognitive function is the executive function.

7. Use of a according to claim 1, wherein the cognitive function is reasoning.

8. A method for improving cognitive functions and performances in normal healthy mammals, wherein an effective amount of a composition comprising L-lysine is administered to a normal healthy mammal to improve the cognitive functions and performances in the normal healthy mammal.

9. A method according to claim 8, wherein the mammal is a human.

10. A method according to claim 8, wherein the cognitive function is pre-attentive and attentive information processing.

11. A method according to claim 8, wherein the cognitive function is attentional capacity.

12. A method according to claim 8, wherein the cognitive function is the memory, including the long-term memory, short-term memory and the working memory.

13. A method according to claim 8, wherein the cognitive function is the executive function.

14. A method according to claim 8, wherein the cognitive function is reasoning.

**Patentansprüche**

1. Verwendung einer Zusammensetzung, die L-Lysin umfasst, für die Verbesserung kognitiver Funktionen und Leistungen bei normalen gesunden Säugern.

**2.** Verwendung nach Anspruch 1, wobei der Säuger ein Mensch ist.

**3.** Verwendung nach Anspruch 1, wobei die kognitive Funktion präattentive und attentive Informationsverarbeitung ist.

**4.** Verwendung nach Anspruch 1, wobei die kognitive Funktion Aufmerksamkeitskapazität ist.

**5.** Verwendung nach Anspruch 1, wobei die kognitive Funktion das Gedächtnis, einschließlich Langzeitgedächtnis, Kurzzeitgedächtnis und Arbeitsgedächtnis, ist.

**6.** Verwendung nach Anspruch 1, wobei die kognitive Funktion die ausführende Funktion ist.

**7.** Verwendung nach Anspruch 1, wobei die kognitive Funktion Denken ist.

**8.** Verfahren zur Verbesserung kognitiver Funktionen und Leistungen bei normalen gesunden Säugern, bei dem eine wirksame Menge einer Zusammensetzung, die L-Lysin umfasst, an einen normalen gesunden Säuger verabreicht wird, um die kognitiven Funktionen und Leistungen bei dem normalen gesunden Säuger zu verbessern.

**9.** Verfahren nach Anspruch 8, wobei der Säuger ein Mensch ist.

**10.** Verfahren nach Anspruch 8, wobei die kognitive Funktion präattentive und attentive Informationsverarbeitung ist.

**11.** Verfahren nach Anspruch 8, wobei die kognitive Funktion Aufmerksamkeitskapazität ist.

**12.** Verfahren nach Anspruch 8, wobei die kognitive Funktion das Gedächtnis, einschließlich Langzeitgedächtnis, Kurzzeitgedächtnis und Arbeitsgedächtnis, ist.

**13.** Verfahren nach Anspruch 8, wobei die kognitive Funktion die ausführende Funktion ist.

**14.** Verfahren nach Anspruch 8, wobei die kognitive Funktion Denken ist.

**Revendications**

**1.** Utilisation d'une composition comprenant de la L-lysine pour l'amélioration des fonctions cognitives et des performances chez des mammifères sains normaux.

**2.** Utilisation suivant la revendication 1, dans laquelle le mammifère est un être humain.

**3.** Utilisation suivant la revendication 1, dans laquelle la fonction cognitive est le traitement pré-attentif et attentif d'information.

**4.** Utilisation suivant la revendication 1, dans laquelle la fonction cognitive est la capacité d'attention.

**5.** Utilisation suivant la revendication 1, dans laquelle la fonction cognitive est la mémoire, comprenant la mémoire à long terme, la mémoire à court terme et la mémoire de travail.

**6.** Utilisation suivant la revendication 1, dans laquelle la fonction cognitive est la fonction d'exécution.

**7.** Utilisation suivant la revendication 1, dans laquelle la fonction cognitive est le raisonnement.

**8.** Procédé pour améliorer les fonctions cognitives et les performances chez des mammifères sains normaux, dans lequel une quantité efficace d'une composition comprenant de la L-lysine est administrée à un mammifère sain normal pour améliorer les fonctions cognitives et les performances chez le mammifère sain normal.

**9.** Procédé suivant la revendication 8, dans lequel le mammifère est un être humain.

**10.** Procédé suivant la revendication 8, dans lequel la fonction cognitive est le traitement pré-attentif et attentif d'information.

**11.** Procédé suivant la revendication 8, dans lequel la fonction cognitive est la capacité d'attention.

**12.** Procédé suivant la revendication 8, dans lequel la fonction cognitive est la mémoire, comprenant la mémoire à long terme, la mémoire à court terme et la mémoire de travail.

**13.** Procédé suivant la revendication 8, dans lequel la fonction cognitive est la fonction d'exécution.

**14.** Procédé suivant la revendication 8, dans lequel la fonction cognitive est le raisonnement.

Figure 1A

Figure 1B

Figure 2

Figure 3A

Figure 3B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0207768 A
- US 2004005311 A
- WO 9516661 A
- EP 0198508 A
- US 2007036870 A
- WO 9833498 A

**Non-patent literature cited in the description**

- **PÅLSSON et al.** *Psychopharmacology (Berl),* May 2007, vol. 192 (1), 9-15